# EUROPEAN PATENT SPECIFICATION

(11) **EP 1 749 126 B1**
(45) Date of publication and mention of the grant of the patent: **10.08.2011**
(21) Application number: 05750119.9
(22) Date of filing: 20.05.2005
(51) Int. Cl.: D04H 1/46

(54) **ABSORBENT TEXTILE PRODUCT**
SAUGFÄHIGER TEXTILARTIKEL
PRODUIT TEXTILE ABSORBANT

(30) Priority: 28.05.2004 IT MI20041076
(43) Date of publication of application: 07.02.2007
(73) Proprietor: Ahlstrom Corporation, 00180 Helsinki (FI)
(72) Inventor: ORLANDI, Vittorio, I-21010 Arsago Seprio (IT)
(74) Representative: Long, Giorgio
(86) International application number: PCT/IT2005/000289
(87) International publication number: WO 2005/116317

(56) References cited:
- WO-A-03/000975
- US-A- 4 818 594
- US-A- 4 959 894
- US-A- 5 405 650

## Description

. The present invention reflates to the field of non-woven textile products, and is applied to the manufacture of absorbent cloths for household cleaning and personal hygiene purposes.

. Absorbent textile products are normally made from cellulose-based raw materials, such as viscose, cotton and the like, which are "naturally" provided with more or less absorbent capacity. However, these raw materials are known to be expensive. In order to reduce the cost of the finished product, efforts have been made to obtain absorbent tissues from synthetic fibers; in this case, however, in order to obtain a finished absorbent product, a treatment is required with specific chemical additives which can provide a "wettable" product. This type of treatment is not permanent, since this tissue tends to become again water-resistant after it has been wet with water and wrung for the first time. Accordingly, the use of these textile products is restricted to disposable applications. Furthermore, besides having a common language, these methods partially improved only the wettability, but not other characteristics such as the overall softness of the tissue.

US 4,818,594 discloses a non-woven fabric produced by blow-spinning a melt or a solution of a spinnable polymeric material to give fibers and filaments which are transformed into a nonwoven fabric by spun depositing them on a receiving means. The non woven fabric is consolidated by means of water-jets and during or immediately after this water-jets consolidation a surfactant is applied as a wetting agent. The nonwoven fabric is subsequently dried.

WO 03/000975 describes a soap-bar made of hydroentangled nonwoven web preferably comprising staple length textile fibers made of 100% polyester. In order to impart elastic characteristics to the nonwoven fabric a polymeric binder composition is uniformly applied to the nonwoven fabric. The binder is preferably acrylic binder. The fabric is also treated with a wetting agent (Tween 20).

However, none of the above documents describe a nonwoven fabric as defined in the characterising portion of claim 1.

. An object of the present invention is to provide a novel textile product consisting of synthetic fibers, which is able to maintain a high absorbing power upon use, by having been treated with a suitable wetting agent.

. A further object of the invention is to provide a non-woven fabric with improved wettability, which has improved characteristics of absorption time, amount of absorbed liquid, capacity of retaining the absorbed liquid and overall softness.

. Now, the Applicant has found that, contrary to those hydro-entangled textile products made of synthetic fibers of the prior art, the treatment with wetting additives being carried out on hydro-entangled spunlace, non-woven fabric as definied in claim 1 surprisingly gives the product high absorption features, which are associated with an improvement in the characteristic spunlace overall softness. These high characteristics persist in the product throughout the cycle of use.

. Similar results have been obtained by carrying out the wetting treatment directly upon manufacture of the non-woven fabric, rather than the finished tissue, particularly between the hydro-entanglement step and the final winding of the product.

. Accordingly, an object of the present invention is a spunlace non-woven textile product as definied in claim 1 consisting of hydro-entangled synthetic fibers that are made permeable through a wetting treatment.

. A further object of the invention is the process for preparing said textile product.

. The inventive textile product can be mainly applied in the household cleaning and personal hygiene field. Particularly, it is adapted for manufacturing cloths, rags and similar fabrics for wiping any surface, wipes, baby wipes, etc.

. The method for preparing this non-woven fabric is carried out according to the technique known as "spunlace", followed by hydro-entanglement of the fibers, preferably through high pressurized water jets; then the free water being entrapped among the fibers is eliminated by means of drying.

. The wetting treatment can be also carried out both on previously existing hydro-entangled spunlace, and upon manufacture of the same. The wetting treatment can also be carried out on the non-woven raw fibers that had been previously treated with wetting agents.

. Preferably, the wetting treatment is carried out after hydro-entanglement and before oven-drying.

. The wetting additives are those commonly employed in the field, such as cationic, anionic or non-ionic surfactants, preferably EUCAROL^{®} available from LAMBERTI spa. In the course of testing, this additive exhibited a great efficacy in terms of giving absorption capacity to the final product with a low dosage. In fact, the low dosage addition of additives leads to highly superior quality products as compared with those products obtained with high dosages, as will be discussed in detail below.

. The treatment method can be selected from those used in common practice. They are, *inter alia:*
- Spraying: the product mixture will be sprayed on the fibers in a stage along the manufacturing line which is comprised between the hydro-entanglement and final winding steps;
- plating: the product mixture will be applied by a contact roller spreading the products on the spunlace surface;
- printing: the same as plating, but with a printing machine;
- impregnation; the spunlace, either already built up or in an intermediate step, is dipped in a mixture of selected products, then the excess amount is wrung by means of a mangle or similar systems;
- foam bonding: a mixture of selected products is treated until it is in a foamy physical state and then applied by conventional techniques (e.g., auger filler, knife coating, etc.).
The dosage of the wetting additive changes as a function of the fibers being used, the spunlace non-woven features (density, fiber distribution, etc.) and desired level of absorption (meaning the amount of absorbed, retained water and the absorption time, capillarity).

. Impregnation followed by a squeezing mangle is the preferred treatment method, which is advantageous over the other methods in that it allows the wetting additive to be spread more evenly throughout the fiber portions, even inside the non-woven. In this case, the bath concentration will range between 1 and 10 g/l, preferably 1-5 g/l. For example, if a solution with 1 g/l additive concentration is used, with the non-woven fabric absorption being 100%, a 1% amount of absorbed additive will be obtained.

. The spunlace, after the hydro-entanglement, is passed into a wetting additive bath at the concentrations described above, then sent to the squeezing mangle and subsequently the oven for drying.

. The squeezing mangle is to be set such that the squeezing is calibrated, i.e. the pressure should be such that a very small amount of liquid is left, in order to make the stay in the drying oven as short as possible. At the same time, the pressure should not be as high as to calender the spunlace, which would result in modifying the non-woven fabric architecture. After a number of tests, it has been found that the best results are obtained with pressure values ranging between 4 and 8 bars, preferably 6 bars, when applied to a spunlace dipped in a wetting additive solution at the above concentrations.

. Following the squeezing mangle, the non-woven fabric is sent to the drying oven. The temperature within the oven depends on the speed at which the product passes therethrough, and is to be adjusted such that complete evaporation of the water is ensured. For example, if a product with 55 g/m² grammage is produced at 150 m/min, the temperature within the oven is to be set at about 120°C for complete water evaporation.

. The synthetic fibers that can be used in the preparation of the present textile products are for example fibers of a material selected from the group consisting of polyester, polypropylene, polyamide, acrylic and mixtures thereof, preferably they are 100% polyester or 100% polypropylene.

. Not all the synthetic fibers available on the market can be used for the purposes of the invention. In fact, after a number of tests, it has been found that the quality of the final product, particularly in terms of softness and absorption capacity, depends on the percentage of additives provided on the starting fibers.

. Several types of additives are added to the synthetic fibers by the manufacturers, such as: lubricating additives to give the fibers smoothness and easy processability; antistatic additives to prevent damaging electrostatic currents that may degrade the product, or at worst, reduce the productivity of the machine; hydrophilic additives to give the least affinity to water; anti-foam additives to avoid that foam may be generated upon the hydro-entanglement step.

. The synthetic fibers that can be used for the purposes of the present invention should have a very low percentage of starting additives. For example, a percentage ranging from <0,1% a 0,2%. Examples of these fibers are 100% polyester fibers purchased from Dupont, Montefibre, Hochst, Catalana, ecc.

. Preferably, the inventive non-woven fabric, in addition of being treated with wetting additives, is also treated with anti-foam additives in order to adjust the surface tension of the wetting agent solution.

. Examples of said anti-foam agents are silicone-based additives. The preferred anti-foam agent is HANSA SP^{®} available from HANSA spa.

. The anti-foam agent can be applied to the non-woven fabric using the various treatment methods as described above. Advantageously, the anti-foam agent is applied by impregnation, simultaneously with the wetting agent, i.e. one single bath is prepared containing both the wetting agent and the anti-foam additives. The anti-foam agent solution that is advantageously employed for the inventive product has a concentration ranging between 1 and 7 g/l, preferably about 2 g/l.

. The anti-foam agent serves to adjust the surface tension of the wetting agent, i.e. the capacity of generating foam upon application. Hydrophilic additives are also available, which are little effective though being poorly foam-forming, and hence require to be used at high dosages. As already discussed above, both the starting additives and the hydrophilic and anti-foam additives that are auditioned upon manufacture impair the final quality of the product when they are added at high dosages.

. Therefore, the best choice is that suggested by the present invention, i.e. using fibers with low amounts of starting additives, to which reduced amounts of very foam-forming wetting agent are applied, and simultaneously, also reduced amounts of an anti-foam additive.

. The inventive product comprises 3 to 9 card webs. Witch a number of webs greater than one, a more isotropic textile structure, and accordingly a maximum spatial layout of the fibers is ensured, which translates into a maximum fiber-water contact surface. Thereby, the water droplets are adsorbed by the structure within the small spaces resulting from the random distribution of the fibers. The absorption capacity of the finished product is ensured by the provision of several webs, along with the application of the wetting agent.

. The textile product thus obtained typically has a weight/surface ratio ranging between 30 and 150 g/m².

. The textile product obtained according to the invention is highly hydrophilic, provided with a considerable absorbing power and softness, and good hand property, also persisting after first use. This is contrary to those non hydro-entangled textile products made of synthetic fibers of the prior art.

. Particularly, the inventive product has a lower absorption time, a greater capacity of retaining the same, as well as a greater overall softness.

. The inventive textile product has a considerable versatility of use, and can be generally applied in the household cleaning and personal hygiene fields. It can be used in the manufacture of any type of surface-drying cloth, such as cloths, rags and similar fibers for cleaning floors and other household surfaces, as well as wipes, baby wipes, etc.

. In the course of testing, the cloth prepared as described above exhibited a considerable absorbing power, which has remained quite unchanged upon use (Table 1).

. Particularly, after it has been subjected to the wetting treatment according to the invention, the absorbing power of the inventive product can be compared with that of a product consisting of 50% viscose and 50% polyester, and is high in the product throughout the cycle of use. This result is even more surprising considering that the comparative product contains 50% viscose, which is a naturally hydrophilic fiber.

**Table 1**

| SAMPLE | DRY WEIGHT /g(m²) | WET WEIGHT /g(m²) | ABSORPTION (%) |
|---|---|---|---|
| 100% polyester | | | |
| Non treated | 51 | 399 | 682 |
| Treated 1g/l | 48 | 411 | 756 |
| Treated 2g/l | 47 | 445 | 847 |
| Treated 5g/l | 48 | 473 | 885 |
| VP | | | |
| Non treated | 51 | 1609 | 1094 |

| | | | |
|---|---|---|---|
| VP = 50% viscose and 50% cellulose | | | |

. The assessment of the absorbing power has been carried out in accordance with the EDANA 10.4-02 ABSORPTION standard.

. Furthermore, in comparative tests, the inventive product, at the same thickness, has proved to have an improved strength to longitudinal and transverse load and improved longitudinal and transverse elongation properties as compared with a cloth consisting of a 50% viscose and 50% polyester mixture, such as illustrated in Tables 2 and 3.

**Table 2**

| | Akena P40 | VP 40 |
|---|---|---|
| Thickness (mm) | 0,57 | 0,56 |
| Load L/T (N) | 70/24 | 46/11,5 |
| Elongation L/T (%) | 52/160 | 38/137 |

**Table 3**

| | Akena P50 | VP 50 |
|---|---|---|
| Thickness (mm) | 0,62 | 0,58 |
| Load L/T (N) | 115/31 | 64/16 |
| Elongation L/T (%) | 49/155 | 38/135 |

| | | |
|---|---|---|
| Akena P40 and P50 = 100% polyester inventive cloth having a grammage of 40 g/m² and 50 g/m², respectively. VP40 and VP50 = comparative cloth of 50% viscose and 50% polyester having a grammage of 40 g/m² and 50 g/m², respectively. L/T = longitudinal and transverse. | | |

## Claims

1. A spunlace non-woven fabric textile product consisting of synthetic fibers that are hydro-entangled and made permeable by means of a wetting treatment **characterized in that** the product comprises 3 to 9 card webs.

2. The product according to claim 1, wherein said fibers are fibers of a material selected from the group consisting of polyester, polypropylene, polyamide, acrylic and mixtures thereof.

3. The product according to claims 1-2, having a grammage ranging between 30 and 150 g/m².

4. The product according to claims 1-3, in the form of a cloth, rag or other fabric for wiping surfaces, wipe, baby wipe.

5. The product according to any claim 1 to 4, consisting of 100% polyester or 100% polypropylene synthetic fibers.

6. A process for preparing the textile product as described in claims 1-5, **characterized in that** a spunlace hydro-entangled tissue in the finished state or a spunlace tissue that has already been hydro-entangled and not yet dried. is subjected to a wetting treatment.

7. The process according to claim 6, wherein the starting fibers are 100% polyester or 100% polypropylene and with a content of starting additives of <0,1% to 0,2%.

8. The process according to any claim 6 to 7, wherein the wetting treatment is carried out by means of a technique selected among spraying, plating, printing, impregnation, application of foam.

9. The process according to any claim 6 to 8 wherein said wetting agent is selected from cationic, anionic or non-ionic surfactants.

10. The process according to any claim 6 to 9, wherein the concentration of the wetting agent solution ranges between 1 and 10 g/l, preferably 1 and 5 g/l.

11. The process according to any claim 6 to 10, comprising a treatment step with an anti-foam additive.

12. The process according to claim 11, wherein said anti-foam treatment is carried out simultaneously with the wetting treatment.

13. The process according to claim 11 or 12 wherein said anti-foam agent is a silicone-based anti-foam agent.

14. The process according to any claim 11 to 13 wherein said anti-foam treatment is carried out by means of impregnation by employing a solution of the anti-foam agent at a concentration ranging between 1 and 7 g/l, preferably about 2 g/1.

15. Use of the textile product described in claims 1-5, for manufacturing products used for household cleaning and personal hygiene purposes .

16. Use according to claim 15, wherein said product is selected from a cloth, rag or other fabric for wiping surfaces, wipe, baby wipe.

## Patentansprüche

1. Nicht-gewebtes Spunlace-Faserstoff-Textilprodukt bestehend aus Synthesefasern, die hydroverwoben und permeabel gemacht sind mittels einer Benetzungsbehandlung **dadurch gekennzeichnet, dass** das Produkt 3 bis 9 Faserflore umfasst.

2. Produkt nach Anspruch 1, bei dem die Fasern Fasern aus einem Material sind, das aus der Gruppe bestehend aus Polyester, Polypropylen, Polyamid, Acryl und Mischungen davon ist ausgewählt ist.

3. Produkt nach Ansprüchen 1-2, mit einer Grammatur zwischen 30 und 150 g/m².

4. Produkt nach Ansprüchen 1-3, in Gestalt eines Tuches, eines Lumpens oder anderen Stoffs zum Wischen von Flächen, eines Wischtuchs, Baby-Wischtuchs.

5. Produkt nach einem der Ansprüche 1 bis 4, das aus 100 % Polyester- oder 100 % Polypropylen-Synthesefasern besteht.

6. Verfahren zur Herstellung eines Textilprodukts nach Ansprüchen 1-5, **dadurch gekennzeichnet, dass** ein Spunlace-hydroverwobenes-Gewebe im Endzustand oder ein Spunlace-Gewebe, dass bereits hydroverwoben und noch nicht getrocknet ist, einer Benetzungsbehandlung unterzogen wird.

7. Verfahren nach Anspruch 6, bei dem die Ausgangsfasern aus 100% Polyester oder 100% Polypropylen bestehen und einen Gehalt an Ausgangszusätzen von < 0,1% bis 0,2% aufweisen.

8. Verfahren nach einem der Ansprüche 6 bis 7, bei dem die Benetzungsbehandlung mittels einer Technik durchgeführt wird, die aus Sprühen, Plattieren, Bedrucken, Imprägnieren, Aufbringen von Schaum ausgewählt ist.

9. Verfahren nach einem der Ansprüche 6 bis 8, bei dem das Benetzungsmittel aus kationischen, anionischen oder nicht-ironischen grenzflächenaktiven Stoffen ausgewählt ist.

10. Verfahren nach einem der Ansprüche 6 bis 9, bei dem die Konzentration der Benetzungsmittellösung zwischen 1 und 10 g/l liegt, vorzugsweise 1 und 5 g/l.

11. Verfahren nach einem der Ansprüche 6 bis 10, mit einem Behandlungsschritt mit einem Antischaumzusatz.

12. Verfahren nach Anspruch 11, bei dem die Antischaum-Behandlung gleichzeitig mit der Benetzungsbehandlung durchgeführt wird.

13. Verfahren nach Anspruch 11 oder 12, bei dem das Antischaummittel ein silikon-basiertes Antischaummittel ist.

14. Verfahren nach einem der Ansprüche 11 bis 13, bei dem die Antischaum-Behandlung mittels Imprägnierung unter Verwendung einer Lösung des Antischaummittels mit einer Konzentration zwischen 1 und 7 g/l durchgeführt wird, vorzugsweise ungefähr 2 g/l.

15. Verwendung eines Textilprodukts nach Ansprüchen 1-5, zur Herstellung von Produkten, die für Haushaltsreinigungs- und Körperpflege-Zwecke verwendet werden.

16. Verwendung nach Anspruch 15, bei der das Produkt aus einem Tuch, einem Lumpen oder einem anderen Stoff zum Reinigen von Flächen, Wischtuch, Baby-Wischtuch ausgewählt ist.

## Revendications

1. Produit textile du type étoffe non tissée lacé par filage, consistant en fibres synthétiques qui sont hydro-enchevêtrées et rendues perméables au moyen d'un traitement de mouillage, ledit produit étant **caractérisé en ce qu'**il comprend 3 à 9 voiles de carde.

2. Produit suivant la revendication 1, dans lequel lesdites fibres sont des fibres d'une matière choisie dans le groupe consistant en un polyester, un polypropylène, un polyamide, une matière acrylique et leurs mélanges.

3. Produit suivant les revendications 1 et 2, ayant un grammage allant de 30 à 150 g/m².

4. Produit suivant les revendications 1 à 3, sous forme d'un torchon, d'un chiffon ou d'une autre étoffe pour essuyer des surfaces, d'un mouchoir ou d'une lingette pour bébé.

5. Produit suivant l'une quelconque des revendications 1 à 4, consistant en fibres synthétiques 100 % polyester ou 100 % polypropylène.

6. Procédé pour la préparation du produit textile tel que décrit dans les revendications 1 à 5, **caractérisé en ce qu'**un tissu hydro-enchevêtré lacé par filage à l'état fini ou un tissu lacé par filage qui a déjà été hydro-enchevêtré et qui n'a pas été encore séché est soumis à un traitement de mouillage.

7. Procédé suivant la revendication 6, dans lequel les fibres de départ sont des fibres 100 % polyester ou 100 % polypropylène, avec une teneur en additifs de départ de moins de 0,1 % à 0,2 %.

8. Procédé suivant l'une quelconque des revendications 6 et 7, dans lequel le traitement de mouillage est effectué au moyen d'une technique choisie entre la pulvérisation, le placage, l'impression, l'imprégnation, l'application d'une mousse.

9. Procédé suivant l'une quelconque des revendications 6 à 8, dans lequel ledit agent mouillant est choisi entre des agents tensioactifs cationiques, anioniques et non ioniques.

10. Procédé suivant l'une quelconque des revendications 6 à 9, dans lequel la concentration de la solution en agent mouillant est comprise entre 1 et 10 g/l, de préférence 1 et 5 g/l.

11. Procédé suivant l'une quelconque des revendications 6 à 10, comprenant une étape de traitement avec un additif antimousse.

12. Procédé suivant la revendication 11, dans lequel ledit traitement antimousse est effectué simultanément avec le traitement mouillant.

13. Procédé suivant la revendication 11 ou 12, dans lequel ledit agent antimousse est un agent antimousse à base de silicone.

14. Procédé suivant l'une quelconque des revendications 11 à 13, dans lequel ledit traitement antimousse est effectué au moyen d'une imprégnation en utilisant une solution de l'agent antimousse à une concentration de 1 à 7 g/l, de préférence d'environ 2 g/l.

15. Utilisation du produit textile décrit dans les revendications 1 à 5 pour la fabrication de produits utilisés pour le nettoyage domestique et à des fins d'hygiène individuelle.

16. Utilisation suivant la revendication 15, dans lequel ledit produit est choisi entre un torchon, un chiffon ou une autre étoffe pour essuyer des surfaces, un mouchoir et une lingette pour bébé.
